# EUROPEAN PATENT APPLICATION

(11) **EP 4 473 835 A1**
(43) Date of publication of application: **11.12.2024**
(21) Application number: 23746774.1
(22) Date of filing: 18.01.2023
(51) Int. Cl.: A01N 33/12, A01N 25/02, A01N 31/02, A01P 1/00, A01P 3/00, A61L 2/18

(54) **COMPOSITION FOR INACTIVATING VIRUS OR BACTERIUM**

(30) Priority: 31.01.2022 JP 2022012774; 07.09.2022 JP 2022141822
(71) Applicant: Kao Corporation, Tokyo 103-8210 (JP)
(72) Inventor: ICHIHASHI Haruna, Wakayama-shi, Wakayama 640-8580 (JP); SUGAHARA Tadashi, Wakayama-shi, Wakayama 640-8580 (JP); YUBUTA Ayaka, Wakayama-shi, Wakayama 640-8580 (JP); YAMAGUCHI Yumi, Wakayama-shi, Wakayama 640-8580 (JP); TABUCHI Yukiko, Wakayama-shi, Wakayama 640-8580 (JP); MINOWA Yu, Wakayama-shi, Wakayama 640-8580 (JP); ISHIHARA Yasuhiro, Wakayama-shi, Wakayama 640-8580 (JP)
(74) Representative: Hoffmann Eitle
(86) International application number: PCT/JP2023/001291
(87) International publication number: WO 2023/145560

(57) **Abstract**

Provided are a viral or bacterial inactivation agent composition and a method for inactivating a virus or a bacterium that have high inactivation effects on viruses and bacteria even under the condition of using a quaternary ammonium salt-type surfactant at a low concentration.

A viral or bacterial inactivation agent composition containing, (A) a quaternary ammonium salt-type surfactant, (B) an aliphatic alcohol with 8 or more and 24 or less carbons having a branched alkyl group or a linear alkenyl group and water.

## Description

### Field of the Invention

The present invention relates to a viral or bacterial inactivation agent composition, and a method for inactivating a virus or a bacterium.

### Background of the Invention

Quaternary ammonium salt-type surfactants are widely used as a base agent for inactivating viruses or bacteria. On the other hand, quaternary ammonium salt-type surfactants are highly toxic to aquatic life, and thus, their mass use or disposal has been cited as a major issue. Further, also for suppressing base agent costs, there is a need for a technology that can achieve high performance at a low concentration. Further, achievement of a reduced formulation concentration of a quaternary ammonium salt-type surfactant in a treatment liquid makes it possible to propose the formulation of a simple and safe treatment liquid which does not need to be rinsed, having big advantages also in terms of product development.

US-B 6143703 discloses an aqueous concentrated liquid disinfectant composition comprising, a botanical oil constituent, a germicidal cationic surfactant having germicidal properties, an organic solvent constituent, a botanical oil solubilizing surfactant selected from alkylpolyoxycarboxylates and alkylarylpolyoxycarboxylates, and a biphenyl solvent constituent.

JP-A 2020-524201 discloses an improved chemical formulation comprising, a quaternary ammonium salt-type surfactant and lauryl alcohol.

CN-A 105767049 discloses a disinfectant liquid for animals comprising, benzalkonium bromide and lauryl alcohol.

CN-A 112335655 discloses an environmentally-friendly complex bactericide comprising, an alkyldimethylbenzylammonium chloride and a long-chain fatty alcohol.

EP-B 2542211 discloses an anti-bacterial oil-in-water-type emulsion comprising, didecyldimethylammonium chloride and an aliphatic alcohol with 12 to 22 carbons.

### Summary of the Invention

The present invention provides a viral or bacterial inactivation agent composition and a method for inactivating a virus or a bacterium that have high inactivation effects on viruses and bacteria even under the condition of using a quaternary ammonium salt-type surfactant at a low concentration.

Here, the inactivation effects on viruses or bacteria refer to partially or totally killing viruses or bacteria to make the viruses or bacteria lose their activity such as infectivity, toxicity or the like.

The present invention relates to a viral or bacterial inactivation agent composition containing, (A) a quaternary ammonium salt-type surfactant (hereinafter referred to as component (A)), (B) an aliphatic alcohol with 8 or more and 24 or less carbons having a branched alkyl group (hereinafter referred to as component (B)) and water.

Further, the present invention relates to a method for inactivating a virus or a bacterium present on a surface of interest including, bringing the viral or bacterial inactivation agent composition of the present invention or a treatment liquid obtained by diluting the same with water into contact with the surface of interest.

According to the present invention, provided are a viral or bacterial inactivation agent composition and a method for inactivating a virus or a bacterium that have high inactivation effects on viruses and bacteria even under the condition of using a quaternary ammonium salt-type surfactant at a low concentration.

### Embodiments of the Invention

The reason why the viral or bacterial inactivation agent composition and the method for inactivating a virus or a bacterium of the present invention have high inactivation effects on viruses and bacteria even under the condition of using a quaternary ammonium salt-type surfactant, component (A) of the present invention, at a low concentration is not wholly certain, but it is inferred to be as follows.

When the viral or bacterial inactivation agent composition of the present invention is brought into contact with the surface of interest on which a virus or a bacterium is present, a specific aliphatic alcohol, component (B) of the present invention, adsorbs to the virus or bacterium prior to a quaternary ammonium salt-type surfactant, component (A), to make the virus or bacterium hydrophobic. It is considered that, as the virus or bacterium is made to be hydrophobic, an adsorption amount of the quaternary ammonium salt-type surfactant is increased, and an inactivation effect is enhanced.

### [Viral or bacterial inactivation agent composition]

### <Component (A)>

The viral or bacterial inactivation agent composition of the present invention contains a quaternary ammonium salt-type surfactant as component (A) .

Component (A) is preferably one or more selected from (a1) compounds represented by the following general formula (a1) (hereinafter referred to as component (a1)) and (a2) compounds represented by the following general formula (a2) (hereinafter referred to as component (a2)), 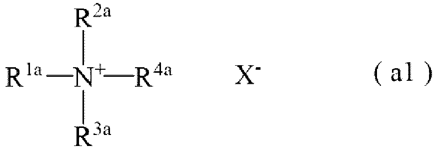
wherein R^{1a} is an aliphatic hydrocarbon group with 8 or more and 18 or less carbons, R^{2a} is a group selected from an aliphatic hydrocarbon group with 8 or more and 18 or less carbons, an alkyl group with 1 or more and 3 or less carbons and a hydroxyalkyl group with 1 or more and 3 or less carbons, R^{3a} and R^{4a} each independently represent a group selected from an alkyl group with 1 or more and 3 or less carbons and a hydroxyalkyl group with 1 or more and 3 or less carbons, and X⁻ is an anion, and 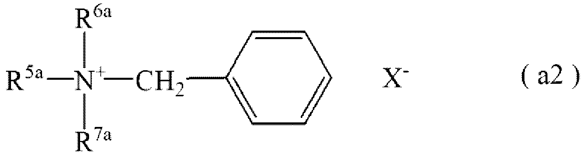
wherein R^{5a} is an aliphatic hydrocarbon group with 8 or more and 18 or less carbons, R^{6a} and R^{7a} each independently represent a group selected from an alkyl group with 1 or more and 3 or less carbons and a hydroxyalkyl group with 1 or more and 3 or less carbons, and X- is an anion.

In the general formula (a1), R^{1a} has 8 or more carbons from the viewpoint of viral or bacterial inactivation performance, and preferably 18 or less, more preferably 14 or less and further preferably 10 or less carbons from the viewpoint of water solubility. R^{1a} is preferably an alkyl group or an alkenyl group and preferably an alkyl group.

In the general formula (a1), R^{2a} is a group selected from an aliphatic hydrocarbon group with 8 or more and 18 or less carbons, an alkyl group with 1 or more and 3 or less carbons and a hydroxyalkyl group with 1 or more and 3 or less carbons.

When R^{2a} is an aliphatic hydrocarbon group with 8 or more and 18 or less carbons, R^{2a} is preferably an alkyl group or an alkenyl group and preferably an alkyl group with preferably 8 or more carbons from the viewpoint of viral or bacterial inactivation performance, and with preferably 18 or less, more preferably 14 or less and further preferably 10 or less carbons from the viewpoint of water solubility.

In the general formula (a1), R^{3a} and R^{4a} each independently represent a group selected from an alkyl group with 1 or more and 3 or less carbons and a hydroxyalkyl group with 1 or more and 3 or less carbons. R^{3a} and R^{4a} preferably each independently represent a group selected from an alkyl group with 1 or more and 3 or less carbons. Examples of the alkyl group with 1 or more and 3 or less carbons include a methyl group, an ethyl group and a propyl group. Examples of the hydroxyalkyl group with 1 or more and 3 or less carbons include a hydroxymethyl group, a hydroxyethyl group and a hydroxypropyl group.

In the general formula (a1), X- is an anion. Examples of the anion include halogen ions, for example, a chloride ion, a bromide ion and an iodide ion. Further, examples include alkyl sulfate ions with 1 or more and 3 or less carbons, for example, a methyl sulfate ion, an ethyl sulfate ion and a propyl sulfate ion.

Examples of a preferable compound of the compounds of the general formula (a1) include one or more selected from an N-alkyl-N,N,N-trimethylammonium salt having an alkyl group with 12 or more and 18 or less carbons, an N,N-dialkyl-N,N-dimethyl-ammonium salt having an alkyl group with 8 or more and 16 or less carbons and an N-alkyl-N,N-dimethyl-N-ethylammonium salt having an alkyl group with 12 or more and 16 or less carbons.

In the general formula (a2), R^{5a} is an aliphatic hydrocarbon group with 8 or more and 18 or less carbons. R^{5a} has preferably 8 or more, more preferably 10 or more and further preferably 12 or more, and preferably 18 or less and more preferably 16 or less carbons from the viewpoint of viral or bacterial inactivation performance. R^{5a} is preferably an alkyl group or an alkenyl group and preferably an alkyl group.

In the general formula (a2), R^{6a} and R^{7a} each independently represent a group selected from an alkyl group with 1 or more and 3 or less carbons and a hydroxyalkyl group with 1 or more and 3 or less carbons. R^{6a} and R^{7a} preferably each independently represent a group selected from an alkyl group with 1 or more and 3 or less carbons. Examples of the alkyl group with 1 or more and 3 or less carbons include a methyl group, an ethyl group and a propyl group. Examples of the hydroxyalkyl group with 1 or more and 3 or less carbons include a hydroxymethyl group, a hydroxyethyl group and a hydroxypropyl group.

In the general formula (a2), X⁻ is an anion. Examples of the anion include halogen ions, for example, a chloride ion, a bromide ion and an iodide ion. Further, examples include alkyl sulfate ions with 1 or more and 3 or less carbons, for example, a methyl sulfate ion, an ethyl sulfate ion and a propyl sulfate ion.

Specific examples of the compounds of the general formula (a2) include one or more compounds selected from an N-octyl-N,N-dimethyl-N-benzyl ammonium salt, an N-decyl-N,N-dimethyl-N-benzyl ammonium salt, an N-dodecyl-N,N-dimethyl-N-benzyl ammonium salt, an N-tridecyl-N,N-dimethyl-N-benzyl ammonium salt, an N-tetradecyl-N,N-dimethyl-N-benzyl ammonium salt, an N-pentadecyl-N,N-dimethyl-N-benzyl ammonium salt, an N-hexadecyl-N,N-dimethyl-N-benzyl ammonium salt, an N-dodecyl-N,N-diethyl-N-benzyl ammonium salt, an N-tridecyl-N,N-diethyl-N-benzyl ammonium salt, an N-tetradecyl-N,N-diethyl-N-benzyl ammonium salt, an N-pentadecyl-N,N-diethyl-N-benzyl ammonium salt, an N-hexadecyl-N,N-diethyl-N-benzyl ammonium salt, an N-dodecyl-N-methyl-N-ethyl-N-benzyl ammonium salt, an N-tridecyl-N-methyl-N-ethyl-N-benzyl ammonium salt, an N-tetradecyl-N-methyl-N-ethyl-N-benzyl ammonium salt, an N-pentadecyl-N-methyl-N-ethyl-N-benzyl ammonium salt and an N-hexadecyl-N-methyl-N-ethyl-N-benzyl ammonium salt.

### <Component (B)>

The viral or bacterial inactivation agent composition of the present invention contains an aliphatic alcohol with 8 or more and 24 or less carbons having a branched alkyl group as component (B).

The aliphatic alcohol of component (B) has 8 or more, preferably 12 or more, more preferably 13 or more, further preferably 16 or more and furthermore preferably 18 or more, and 24 or less, preferably 22 or less and more preferably 20 or less carbons from the viewpoints of viral or bacterial inactivation performance and low-temperature stability.

Component (B) is preferably a compound represented by the following general formula (b1) from the viewpoints of viral or bacterial inactivation performance and low-temperature stability, 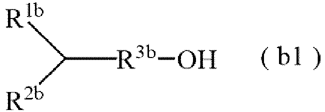 wherein R^{1b} is an alkyl group with 1 or more and 22 or less carbons, R^{2b} is an alkyl group with 1 or more and 22 or less carbons, R^{3b} is an alkylene group with 1 or more and 2 or less carbons, and a total carbon number of R^{1b}, R^{2b} and R^{3b} is 13 or more and 24 or less carbons.

In the general formula (b1), R^{1b} is an alkyl group with 1 or more, preferably 4 or more, preferably 8 or more, more preferably 9 or more and further preferably 10 or more, and 22 or less, preferably 18 or less, more preferably 14 or less and further preferably 12 or less carbons from the viewpoints of viral or bacterial inactivation performance at a low concentration and low-temperature stability. R^{2b} is an alkyl group with 1 or more, preferably 4 or more and more preferably 8 or more, and 22 or less, preferably 18 or less, more preferably 14 or less and further preferably 10 or less carbons from the viewpoint of viral or bacterial inactivation performance at a low concentration. R^{3b} is an alkylene group with 1 or more and 2 or less and preferably one carbon. A total carbon number of R^{1b} and R^{2b} is 11 or more, preferably 13 or more, more preferably 14 or more, further preferably 15 or more, furthermore preferably 16 or more and furthermore preferably 17 or more, and 22 or less, preferably 20 or less and more preferably 18 or less from the viewpoint of viral or bacterial inactivation performance at a low concentration. A total carbon number of R^{1b}, R^{2b} and R^{3b} is 13 or more, preferably 16 or more, more preferably 18 or more and further preferably 19 or more, and 24 or less, preferably 22 or less and more preferably 20 or less from the viewpoint of viral or bacterial inactivation performance at a low concentration.

An aliphatic alcohol with a melting point of preferably 25°C or less, more preferably 10°C or less and further preferably 0°C or less is suitable as component (B) from the viewpoint of storage stability of the treatment liquid.

Specific examples of the aliphatic alcohol having a branched alkyl group of component (B) include one or more selected from branched C13-OH (an aliphatic alcohol with 13 carbons having a branched alkyl group), branched C16-OH (an aliphatic alcohol with 16 carbons having a branched alkyl group, melting point -15°C), branched C15-OH (an aliphatic alcohol with 15 carbons having a branched alkyl group, melting point -26°C), branched C17-OH (an aliphatic alcohol with 17 carbons having a branched alkyl group, melting point -7°C), branched C18-OH (an aliphatic alcohol with 18 carbons having a branched alkyl group, melting point -9°C), branched C19-OH (an aliphatic alcohol with 19 carbons having a branched alkyl group) and branched C20-OH (an aliphatic alcohol with 20 carbons having a branched alkyl group, melting point -7°C), and from the viewpoints of viral or bacterial inactivation performance at a low concentration and base agent availability, one or more selected from branched C16-OH, branched C18-OH and branched C20-OH are preferable, one or more selected from branched C18-OH and branched C20-OH are more preferable, and branched C20-OH is further preferable.

### <Composition and others>

A content of component (A) in the viral or bacterial inactivation agent composition of the present invention is preferably 1 ppm or more, more preferably 5 ppm or more, further preferably 20 ppm or more and furthermore preferably 50 ppm or more from the viewpoint of viral or bacterial inactivation performance, and preferably 5000 ppm or less, more preferably 3000 ppm or less, further preferably 1000 ppm or less, furthermore preferably 300 ppm or less and furthermore preferably 100 ppm or less from the viewpoint of costs.

Note that, in the present invention, a mass of component (A) is specified using a value expressed in terms of a chloride salt.

The viral or bacterial inactivation agent composition of the present invention preferably contains components (a1) and (a2) as component (A) from the viewpoints of viral or bacterial inactivation performance and costs.

When the viral or bacterial inactivation agent composition of the present invention contains components (a1) and (a2), a mass ratio of a content of component (a2) to a content of component (a1), (a2)/(a1), is preferably 0/10 or more, more preferably 1.5/8.5 or more and further preferably 2/8 or more from the viewpoint of costs, and preferably 8/2 or less, more preferably 7/3 or less, further preferably 6/4 or less and furthermore preferably 4/6 or less from the viewpoint of viral or bacterial inactivation performance.

A content of component (B) in the viral or bacterial inactivation agent composition of the present invention is preferably 1 ppm or more, more preferably 2 ppm or more and further preferably 3 ppm or more from the viewpoint of viral or bacterial inactivation performance, and preferably 1500 ppm or less, more preferably 1000 ppm or less, further preferably 500 ppm or less, furthermore preferably 250 ppm or less and furthermore preferably 100 ppm or less from the viewpoint of costs.

The viral or bacterial inactivation agent composition of the present invention can contain an aliphatic alcohol having a linear alkyl group in the range that the effects of the present invention are not impaired.

When the viral or bacterial inactivation agent composition of the present invention contains an aliphatic alcohol having a linear alkyl group, a content of component (B) in all aliphatic alcohols is preferably 50 mass% or more and more preferably 80 mass% or more, and preferably 100 mass% or less.

A mass ratio of a content of component (B) to a content of component (A) in the viral or bacterial inactivation agent composition of the present invention, (B)/(A), is preferably 1/10 or more, more preferably 1/8 or more, further preferably 1/4 or more, furthermore preferably 1/2 or more and furthermore preferably 1/1 or more, and preferably 5/1 or less, more preferably 4/1 or less and further preferably 2/1 or less from the viewpoint of viral or bacterial inactivation performance.

The viral or bacterial inactivation agent composition of the present invention contains water. A content of water in the viral or bacterial inactivation agent composition of the present invention is preferably 5 mass% or more, more preferably 10 mass% or more, further preferably 20 mass% or more, furthermore preferably 50 mass% or more, furthermore preferably 70 mass% or more, furthermore preferably 80 mass% or more and furthermore preferably 90 mass% or more, and preferably 99.999 mass% or less, more preferably 99.99 mass% or less and further preferably 99.95 mass% or less.

The viral or bacterial inactivation agent composition of the present invention can contain (C) a surfactant [excluding component (A)] [hereinafter referred to as component (C)] from the viewpoint of cleaning performance on a surface of interest.

Examples of component (C) include, for example, one or more selected from (C1) an anionic surfactant [hereinafter referred to as component (C1)], (C2) a nonionic surfactant [hereinafter referred to as component (C2)], (C3) a semipolar surfactant [hereinafter referred to as component (C3)] and (C4) an amphoteric surfactant [hereinafter referred to as component (C4)].

Examples of component (C1) include one or more selected from an alkyl sulfate having an alkyl group with 8 or more and 22 or less carbons, an alkyl benzene sulfonic acid having an alkyl group with 8 or more and 22 or less carbons, a polyoxyalkylene alkyl ether sulfate having an alkyl group with 8 or more and 22 or less carbons (in which the oxyalkylene group is an oxyalkylene group with 2 or 3 carbons and preferably an oxyethylene group, and an average number of added moles of the oxyalkylene group is 0.5 or more and 5 or less and preferably 0.5 or more and 3 or less), a fatty acid with 8 or more and 22 or less carbons, and salts of these.

Examples of component (C2) include one or more selected from a polyoxyalkylene alkyl ether having an alkyl group with 8 or more and 22 or less carbons (in which the oxyalkylene group is an oxyalkylene group with 2 or 3 carbons and preferably an oxyethylene group, and an average number of added moles of the oxyalkylene group is 3 or more and 50 or less and preferably 3 or more and 20 or less), an alkyl glucoside having an alkyl group with 8 or more and 22 or less carbons (in which an average degree of condensation of a sugar backbone such as glucose or the like is 1 or more and 5 or less and preferably 1 or more and 2 or less) and a polyoxyalkylene adduct of pentaerythritol (in which the oxyalkylene group is an oxyalkylene group with 2 or 3 carbons and preferably an oxypropylene group, and an average number of added moles of the oxyalkylene group is 3 mol or more and 15 mol or less and preferably 5 mol or more and 8 mol or less).

Examples of component (C3) include an amine oxide-type surfactant having one or more and preferably one alkyl group or alkenyl group with 7 or more and 22 or less carbons.

A compound of the following general formula (c3) is suitable as the amine oxide-type surfactant: 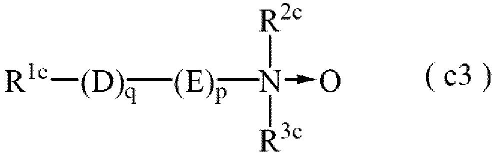 wherein R^{1c} represents an alkyl group or an alkenyl group and more preferably an alkyl group with 7 or more and 22 or less carbons, R^{2c} and R^{3c} are the same or different and represent alkyl groups with 1 or more and 3 or less carbons, D represents -NHC(=O)- group or - C(=O)NH- group, E represents an alkylene group with 1 or more and 5 or less carbons, and q and p represent q = 0 and p = 0 or q = 1 and p =1.

In the above general formula (c3), when q = 1 and p = 1, R^{1c} is a hydrocarbon group and preferably an alkyl group with 9 or more and preferably 11 or more, and 18 or less, preferably 16 or less and more preferably 14 or less carbons from the viewpoint of foamability.

Further, when q = 0 and p = 0, R^{1c} is a hydrocarbon group and preferably an alkyl group with 10 or more and preferably 12 or more, and 18 or less, preferably 16 or less and more preferably 14 or less carbons from the viewpoint of foamability. In the present invention, q = 0 and p = 0 is preferable. R^{2c} and R^{3c} are preferably methyl groups with one carbon.

Examples of component (C4) include a betaine-type surfactant and preferably a sulfobetaine-type surfactant or a carbobetaine-type surfactant having one or more and preferably one alkyl group or alkenyl group with 7 or more and 22 or less carbons.

A compound of the following general formula (c4) is suitable as the betaine-type surfactant: 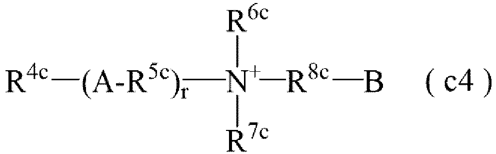 wherein R^{4c} is an alkyl group or an alkenyl group with 7 or more and 18 or less carbons, R^{5c} is an alkylene group with 1 or more and 6 or less carbons, A is a group selected from -COO-, -CONH-, -OCO-, -NHCO- and -O-, r is a number of 0 or 1, R^{6c} and R^{7c} are alkyl groups or hydroxyalkyl groups with 1 or more and 3 or less carbons, R^{8c} is an alkylene group with 1 or more and 5 or less carbons which may be substituted by a hydroxy group, and B is -SO₃⁻, -OSO₃⁻ or -COO⁻.

In the general formula (c4), R^{4c} is an alkyl group or an alkenyl group and preferably an alkyl group with 7 or more and preferably 10 or more, and 18 or less, preferably 14 or less and more preferably 12 or less carbons from the viewpoint of foamability.
A is preferably -COO- or -CONH- and more preferably -CONH-.
R^{5c} preferably has 2 or 3 carbons.
r is preferably 0.
R^{6c} and R^{7c} are preferably methyl groups.
R^{8c} preferably has one carbon.
B is preferably -COO⁻.

Specific examples of the betaine-type surfactant include one or more selected from an alkyl carboxybetaine, an alkyl amide carboxybetaine, an alkyl sulfobetaine, an alkyl hydroxy sulfobetaine, an alkyl amide hydroxy sulfobetaine and an alkyl amino fatty acid salt, and from the viewpoint of foamability, one or more selected from an alkyl amide propyl-N,N-dimethyl carboxybetaine and an alkyl-N,N-dimethyl acetic acid betaine are preferable. The alkyl groups of these have 7 or more and preferably 10 or more, and 18 or less, preferably 14 or less and more preferably 12 or less carbons.

A content of component (C) in the viral or bacterial inactivation agent composition of the present invention is preferably 5 ppm or more, more preferably 10 ppm or more, further preferably 100 ppm or more and furthermore preferably 500 ppm or more, and preferably 30000 ppm or less, more preferably 10000 ppm or less, further preferably 5000 ppm or less, furthermore preferably 2500 ppm or less, furthermore preferably 1000 ppm or less and furthermore preferably 500 ppm or less from the viewpoint of cleaning performance on a surface of interest.

Note that, in the present invention, when component (C1) is used as component (C), a mass of component (C1) is specified using a value expressed in terms of a sodium salt.

When the viral or bacterial inactivation agent composition of the present invention contains component (C1), a content of component (C1) in the viral or bacterial inactivation agent composition of the present invention is preferably 5 ppm or more, more preferably 10 ppm or more, further preferably 20 ppm or more, furthermore preferably 100 ppm or more, furthermore preferably 500 ppm or more and furthermore preferably 800 ppm or more from the viewpoint of foamability, and preferably 10000 ppm or less, more preferably 8000 ppm or less, further preferably 6000 ppm or less, furthermore preferably 4000 ppm or less and furthermore preferably 3000 ppm or less from the viewpoint of bactericidal performance.

When the viral or bacterial inactivation agent composition of the present invention contains component (C2), a content of component (C2) in the viral or bacterial inactivation agent composition of the present invention is preferably 10 ppm or more, more preferably 50 ppm or more, further preferably 200 ppm or more, furthermore preferably 500 ppm or more and furthermore preferably 1000 ppm or more, and preferably 30000 ppm or less, more preferably 10000 ppm or less, further preferably 8000 ppm or less, furthermore preferably 5000 ppm or less and furthermore preferably 4000 ppm or less from the viewpoint of foamability.

When the viral or bacterial inactivation agent composition of the present invention contains component (C3), a content of component (C3) in the viral or bacterial inactivation agent composition of the present invention is preferably 10 ppm or more, more preferably 20 ppm or more, further preferably 100 ppm or more, furthermore preferably 200 ppm or more and furthermore preferably 500 ppm or more, and preferably 10000 ppm or less, more preferably 5000 ppm or less, further preferably 2000 ppm or less, furthermore preferably 1500 ppm or less and furthermore preferably 1000 ppm or less from the viewpoint of foamability.

When the viral or bacterial inactivation agent composition of the present invention contains component (C4), a content of component (C4) in the viral or bacterial inactivation agent composition of the present invention is preferably 10 ppm or more, more preferably 20 ppm or more, further preferably 50 ppm or more, furthermore preferably 300 ppm or more, furthermore preferably 500 ppm or more and furthermore preferably 700 ppm or more, and preferably 30000 ppm or less, more preferably 10000 ppm or less, further preferably 8000 ppm or less, furthermore preferably 5000 ppm or less and furthermore preferably 3000 ppm or less from the viewpoint of foamability.

The viral or bacterial inactivation agent composition of the present invention can contain (D) a water-soluble organic solvent [excluding component (B)] [hereinafter referred to as component (D)] from the viewpoint of base agent solubility.

The water-soluble organic solvent of the present invention refers to a compound which is soluble in an amount of 20 g or more in 100 g of deionized water at 20°C.

Here, the base agent refers to component (A) or (B) .

Examples of component (D) can include one or more selected from (D1) a polyhydric alcohol with 2 or more and 4 or less carbons (hereinafter referred to as component (D1)), (D2) a di or trialkylene glycol having an alkylene glycol unit with 2 or more and 4 or less carbons (hereinafter referred to as component (D2)), (D3) a monoalkoxy (methoxy, ethoxy, propoxy, butoxy) ether or a phenoxy or benzoxy ether of a di to tetraalkylene glycol having an alkylene glycol unit with 2 or more and 4 or less carbons (hereinafter referred to as component (D3)) and (D4) a monohydric alcohol with 1 or more and 3 or less carbons (hereinafter referred to as component (D4)).

Component (D) is preferably one or more selected from components (D1), (D2) and (D3) from the viewpoint of mildness to skin.

Component (D) is preferably a water-soluble organic solvent with 2 or more carbons and preferably 3 or more carbons, and 10 or less carbons and preferably 8 or less carbons.

Specifically, examples of component (D1) include ethylene glycol, propylene glycol, glycerin and isoprene glycol, examples of component (D2) include diethylene glycol and dipropylene glycol, and examples of component (D3) include propylene glycol monomethyl ether, propylene glycol monoethyl ether, diethylene glycol monobutyl ether (which is also referred to as butyldiglycol or the like), phenoxy ethanol, phenoxy triethylene glycol and phenoxy isopropanol, and one or two or more of these can be used. Component (D) is preferably one or more water-soluble organic solvents selected from propylene glycol, dipropylene glycol, diethylene glycol monobutyl ether, phenoxy ethanol, phenyl glycol and phenoxy isopropanol. Component (D) preferably has an alkoxy group, and more preferably is diethylene glycol monobutyl ether.

Note that component (D4) is used as bactericidal or disinfecting alcohols, and attains bactericidal effects when contained at a high concentration, but has the risk of causing skin problems such as rough hands or the like when frequently brought into contact with the human body such as skin or the like, and thus, the viral or bacterial inactivation agent composition of the present invention may be free of component (D4).

A content of component (D4) in the viral or bacterial inactivation agent composition of the present invention is preferably 100000 ppm or less, more preferably 30000 ppm or less, further preferably 10000 ppm or less, furthermore preferably 5000 ppm or less, furthermore preferably 2500 ppm or less, furthermore preferably 1000 ppm or less, furthermore preferably 500 ppm or less, furthermore preferably 250 ppm or less, furthermore preferably 100 ppm or less, furthermore preferably 50 ppm or less, furthermore preferably 10 ppm or less and furthermore preferably 1 ppm or less from the viewpoint of irritation to skin. Further, the viral or bacterial inactivation agent composition of the present invention may be free of an alcohol with 1 or more and 3 or less carbons.

A content of component (D) (excluding component (D4)), particularly components (D1) to (D3) in the viral or bacterial inactivation agent composition of the present invention is preferably 10 ppm or more, more preferably 50 ppm or more and further preferably 100 ppm or more, and preferably 50000 ppm or less, more preferably 10000 ppm or less and further preferably 5000 ppm or less.

The viral or bacterial inactivation agent composition of the present invention can contain (E) an organic acid or a salt thereof [hereinafter referred to as component (E)] from the viewpoint of bactericidal performance in the presence of hardness components.

Examples of component (E) include one or more selected from capric acid, sorbic acid, caproic acid, propionic acid, formic acid, acetic acid, benzoic acid, salicylic acid, lactic acid, tartaric acid, malic acid, glycolic acid, gluconic acid, citric acid, maleic acid, fumaric acid, oxalic acid, adipic acid, glutamic acid, succinic acid, sebacic acid, azelaic acid and salts of these from the viewpoint of bactericidal performance in the presence of hardness components.

Examples of the salt of component (E) include one or more selected from a sodium salt, a potassium salt, a magnesium salt and a calcium salt.

Component (E) is preferably one or more selected from gluconic acid, citric acid and salts of these from the viewpoint of bactericidal performance in the presence of hardness components.

A content of component (E) in the viral or bacterial inactivation agent composition of the present invention is preferably 0.1 ppm or more, more preferably 1 ppm or more, further preferably 5 ppm or more, furthermore preferably 20 ppm or more, furthermore preferably 40 ppm or more and furthermore preferably 80 ppm or more, and preferably 700 ppm or less, more preferably 500 ppm or less and further preferably 350 ppm or less from the viewpoint of bactericidal performance in the presence of hardness components.

Note that, in the present invention, a mass of component (E) is specified using a value expressed in terms of a sodium salt.

The viral or bacterial inactivation agent composition of the present invention has high inactivation effects on viruses and bacteria even if it is free of a bactericidal agent other than component (A).

When the bactericidal agent is used in combination, specific examples of the bactericidal agent include one or more selected from triclosan, isopropyl methylphenol, resorcin, trichlorocarbanilide, chlorhexidine chloride, chlorhexidine gluconate, hydrogen peroxide, povidone iodine and iodine tincture.

A content of a bactericidal agent other than component (A) in the viral or bacterial inactivation agent composition of the present invention is preferably 1000 ppm or less, more preferably 100 ppm or less, further preferably 50 ppm or less, furthermore preferably 10 ppm or less, furthermore preferably 1 ppm or less and furthermore preferably 0.5 ppm or less from the viewpoint of costs. Further, the viral or bacterial inactivation agent composition of the present invention may be free of a bactericidal agent other than component (A).

The viral or bacterial inactivation agent composition of the present invention can contain components such as, for example, an inorganic salt, an inorganic acid, a fragrance (natural fragrance, synthetic fragrance), a thickener, a gelling agent, a polymeric compound (dispersant), a hydrotropic agent, a moisture retention agent, a preservative, an antioxidant, a photostabilizer, an antiseptic, a buffer and others (excluding components qualifying as components (A) to (E)) in the range that the effects of the present invention are not impaired.

In the viral or bacterial inactivation agent composition of the present invention, a pH at 25°C measured by a glass electrode method is preferably 3 or more, more preferably 4 or more and further preferably 5 or more, and preferably 10 or less, more preferably 9 or less and further preferably 8 or less from the viewpoint of skin irritation.

The viral or bacterial inactivation agent composition of the present invention can be used, for example, in liquid form, gel form or paste form. The treatment liquid of the present invention can appropriately contain a solubilization carrier such as water, a solvent or the like, a gelling agent, or the like depending on those forms of use.

Examples of viruses to which the viral or bacterial inactivation agent composition of the present invention is directed include enveloped viruses and non-enveloped viruses.

The enveloped viruses refer to a single-stranded (+) RNA virus, a single-stranded (-) RNA virus, a double-stranded RNA virus, a single-stranded DNA virus and a double-stranded DNA virus having a lipid layer or a lipid bilayer on the viral surfaces.

Examples of the enveloped viruses include herpesvirus, influenza virus, paramyxovirus, rabies virus, respiratory syncytial virus, coronavirus, HIV, smallpox virus, hepatitis B virus, hepatitis C virus, hepatitis D virus, rubella virus, SARS coronavirus (SARS-CoV), SARS coronavirus 2 (SARS-CoV-2) and MERS coronavirus (MERS-CoV), phage virus and others.

The non-enveloped viruses refer to viruses other than enveloped viruses, and refer to a single-stranded (+) RNA virus, a single-stranded (-) RNA virus, a double-stranded RNA virus, a single-stranded DNA virus and a double-stranded DNA virus having neither lipid layer nor lipid bilayer on the viral surfaces.

Typical examples of the non-enveloped viruses include adenovirus, parvovirus, papovavirus, human papillomavirus and others as viruses with DNA genome, and rotavirus, coxsackie virus, enterovirus, sapovirus, norovirus, poliovirus, echovirus, hepatitis A virus, hepatitis E virus, rhinovirus, astrovirus and others as viruses with RNA genome.

Examples of bacteria to which the viral or bacterial inactivation agent composition of the present invention is directed include molds, typically fungi of the genus *Cladosporium,* the genus *Aspergillus,* the genus *Candida,* the genus *Penicillium,* the genus *Alternaria,* the genus *Phoma* and the genus *Aureobasidium,* bacteria of the genus *Moraxella* such as *Moraxella sp., Moraxella osloensis* and others, bacteria of the genus *Micrococcus* such as *Micrococcus luteus, Micrococcus lylae, Micrococcus aloeverae* and others, fungi of the genus *Saccharomyces,* the genus *Rhodotorula* and the genus *Pichia,* bacteria of the genus *Pseudomonas* such as *Pseudomonas aeruginosa, Pseudomonas putida* and others, gram-negative bacteria such as *Escherichia coli, Alcaligenes faecalis, Klebsiella pneumoniae, Proteus vulgaris, Serratia marcescens, Methylobacterium* and others, and gram-positive bacteria, typically *Staphylococcus aureus.*

The viral or bacterial inactivation agent composition of the present invention is directed to enveloped viruses or gram-negative bacteria, and specifically a virus or a bacterium selected from influenza virus, coronavirus, *Escherichia coli, Klebsiella, Moraxella osloensis, Micrococcus luteus* and *Pseudomonas aeruginosa,* particularly a virus or a bacterium selected from influenza virus and *Escherichia coli,* and has high inactivation effects on these bacteria or viruses.

Uses of the viral or bacterial inactivation agent composition of the present invention can cover (1) use on a hard article, (2) use on a livestock animal or livestock breeding equipment, or the like.

Examples of the hard article include a hard article having a hard surface such as, for example, a bathroom, a toilet, a kitchen, a floor, a doorknob, tableware, food processing equipment, a desk, a chair, a wall or the like. These hard articles may be those used in households or those used in public facilities or plants, for example, a pool, a bathhouse, a dining lounge, a hospital and others.

Examples of the livestock animal include cattle, pigs, birds or the like.

Examples of the livestock breeding equipment include a floor surface, a wall surface or an entrance and exit of a livestock shed such as a cowshed, a pigsty, a bird cage or the like, an article set or placed within those facilities, and a tooling or machinery used for work carried out within those facilities.

### [Method for inactivating virus or bacterium]

The present invention provides a method for inactivating a virus or a bacterium present on a surface of interest including, bringing the viral or bacterial inactivation agent composition of the present invention or a treatment liquid obtained by diluting the same with water (hereinafter referred to as the treatment liquid of the present invention) into contact with the surface of interest.

The aspects described in the viral or bacterial inactivation agent composition of the present invention can be appropriately applied to the method for inactivating a virus or a bacterium of the present invention.

Viruses or bacteria to which the method for inactivating a virus or a bacterium of the present invention is directed are the same as those described in the viral or bacterial inactivation agent composition of the present invention.

Examples of the surface of interest which is brought into contact with the viral or bacterial inactivation agent composition of the present invention or the treatment liquid of the present invention in the method for inactivating a virus or a bacterium of the present invention include (1) a surface of a hard article and (2) a surface of a livestock animal or livestock breeding equipment.

Hard articles and livestock animals or livestock breeding equipment to which the method for inactivating a virus or a bacterium of the present invention is directed are the same as those described in the viral or bacterial inactivation agent composition of the present invention.

A dilution ratio when the viral or bacterial inactivation agent composition of the present invention is diluted with water to prepare the treatment liquid of the present invention is preferably 1000 times or less, more preferably 100 times or less and further preferably 10 times or less from the viewpoint of viral or bacterial inactivation performance.

In the treatment liquid of the present invention, a pH at 25°C measured by a glass electrode method is preferably 3 or more, more preferably 4 or more and further preferably 5 or more, and preferably 10 or less, more preferably 9 or less and further preferably 8 or less from the viewpoint of skin irritation.

Examples of a method for bringing the viral or bacterial inactivation agent composition of the present invention or the treatment liquid of the present invention into contact with the surface of interest on which a virus or a bacterium is present include a method for spraying or applying the viral or bacterial inactivation agent composition of the present invention or the treatment liquid of the present invention to the surface of interest on which a virus or a bacterium is present or considered to be present, or a method for immersing the surface of interest in the viral or bacterial inactivation agent composition of the present invention or the treatment liquid of the present invention. Further, non-woven fabric may be impregnated with the viral or bacterial inactivation agent composition of the present invention or the treatment liquid of the present invention and brought into contact with the surface of interest.

When the viral or bacterial inactivation agent composition of the present invention or the treatment liquid of the present invention is sprayed or applied to the surface of interest, the viral or bacterial inactivation agent composition of the present invention or the treatment liquid of the present invention may be filled into a container provided with a sprayer and sprayed in liquid droplet form or foam form, or the viral or bacterial inactivation agent composition of the present invention or the treatment liquid of the present invention may be poured from a container onto the surface of interest and applied with a brush or the like.

Examples of the container provided with a sprayer include a manual spray device using no propellant, such as a trigger-type spray container, a pump-type spray container or the like, an aerosol using a propellant, and others.

The container provided with a sprayer is preferably a trigger-type spray capable of spraying the contents in liquid droplet form or foam form, and more preferably a trigger-type spray provided with a mechanism for spraying the contents in liquid droplet form, or a trigger-type spray provided with a mechanism for forming foam (foam forming mechanism).

When non-woven fabric is impregnated with the viral or bacterial inactivation agent composition of the present invention or the treatment liquid of the present invention and brought into contact with the surface of interest, non-woven fabric processed into sheet form can be used, and a fiber constituting the non-woven fabric is preferably made of one or more fibers selected from a hydrophilic fiber and a hydrophobic fiber.

In the present invention, the hydrophilic fiber refers to a fiber whose moisture content under standard conditions (20°C, 65%RH) is more than 5 mass%. Note that the moisture content under standard conditions is measured by a method specified in JIS L 1013 or JIS L 1015. Further, the hydrophobic fiber refers to a fiber whose moisture content under standard conditions (20°C, 65%RH) is 5 mass% or less.

A method for contacting with the surface of interest may be contacting by pressing the non-woven fabric impregnated with the viral or bacterial inactivation agent composition of the present invention or the treatment liquid of the present invention onto the surface of interest, and applying external force in the range that an object is not damaged, thereby transferring the viral or bacterial inactivation agent composition of the present invention or the treatment liquid of the present invention with which the non-woven fabric is impregnated to the surface of interest, and the applying external force may be any of scrubbing, rubbing and tapping.

A use amount of the viral or bacterial inactivation agent composition of the present invention or the treatment liquid of the present invention is not particularly limited, but can be, for example, in terms of an amount of component (A) per cm² of a unit area of an object, preferably 0.05 µg/cm² or more and more preferably 1 µg/cm² or more from the viewpoint of viral or bacterial inactivation performance, and preferably 5 mg/cm² or less and more preferably 1 mg/cm² or less from the viewpoint of costs.

A period of time during which the viral or bacterial inactivation agent composition of the present invention or the treatment liquid of the present invention is brought into contact with the surface of interest on which a virus or a bacterium of interest is present (a period of time during which they are left alone) is preferably 10 seconds or more, more preferably 1 minute or more and further preferably 5 minutes or more, and preferably 60 minutes or less, more preferably 30 minutes or less and further preferably 20 minutes or less from the viewpoint of viral or bacterial inactivation performance.

After brought into contact, it may be dried as-is, wiped off with clean cloth or the like, or rinsed with water. When it is rinsed, external force (physical force) may be applied with a sponge or the like, or it may be simply rinsed with a stream of water.

In addition to the aforementioned embodiments, the present invention discloses the aspects below.
<1> A viral or bacterial inactivation agent composition containing, (A) a quaternary ammonium salt-type surfactant (hereinafter referred to as component (A)), (B) an aliphatic alcohol with 8 or more and 24 or less carbons having a branched alkyl group (hereinafter referred to as component (B)) and water.
<2> The viral or bacterial inactivation agent composition according to <1>, wherein component (A) is one or more selected from compounds represented by the following general formula (a1) and compounds represented by the following general formula (a2), 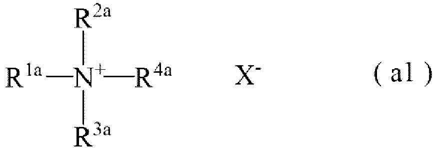
   wherein R^{1a} is an aliphatic hydrocarbon group with 8 or more and 18 or less carbons, R^{2a} is a group selected from an aliphatic hydrocarbon group with 8 or more and 18 or less carbons, an alkyl group with 1 or more and 3 or less carbons and a hydroxyalkyl group with 1 or more and 3 or less carbons, R^{3a} and R^{4a} each independently represent a group selected from an alkyl group with 1 or more and 3 or less carbons and a hydroxyalkyl group with 1 or more and 3 or less carbons, and X- is an anion, and 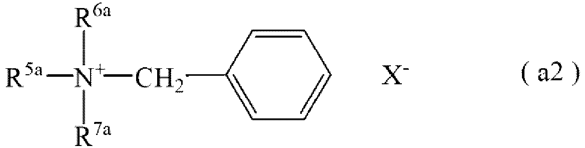
   wherein R^{5a} is an aliphatic hydrocarbon group with 8 or more and 18 or less carbons, R^{6a} and R^{7a} each independently represent a group selected from an alkyl group with 1 or more and 3 or less carbons and a hydroxyalkyl group with 1 or more and 3 or less carbons, and X⁻ is an anion.
<3> The method for inactivating a virus or a bacterium according to <2>, wherein in the general formula (a1), R^{1a} is an alkyl group or an alkenyl group and preferably an alkyl group with 8 or more, and preferably 18 or less, more preferably 14 or less and further preferably 10 or less carbons, R^{2a} is a group selected from an aliphatic hydrocarbon group with 8 or more and 18 or less carbons, an alkyl group with 1 or more and 3 or less carbons and a hydroxyalkyl group with 1 or more and 3 or less carbons, and is an alkyl group or an alkenyl group and preferably an alkyl group with preferably 8 or more, and preferably 18 or less, more preferably 14 or less and further preferably 10 or less carbons, R^{3a} and R^{4a} each independently represent a group selected from an alkyl group with 1 or more and 3 or less carbons and a hydroxyalkyl group with 1 or more and 3 or less carbons, and preferably an alkyl group with 1 or more and 3 or less carbons, and X⁻ is an anion, preferably an anion selected from a halogen ion and an alkyl sulfate ion with 1 or more and 3 or less carbons and more preferably an anion selected from a chloride ion, a bromide ion, an iodide ion, a methyl sulfate ion, an ethyl sulfate ion and a propyl sulfate ion.
<4> The method for inactivating a virus or a bacterium according to <2> or <3>, wherein in the general formula (a2), R^{5a} is an alkyl group or an alkenyl group and preferably an alkyl group with 8 or more, more preferably 10 or more and further preferably 12 or more, and preferably 18 or less and more preferably 16 or less carbons, R^{6a} and R^{7a} preferably each independently represent a group selected from an alkyl group with 1 or more and 3 or less carbons and a hydroxyalkyl group with 1 or more and 3 or less carbons and preferably an alkyl group with 1 or more and 3 or less carbons, and represent an alkyl group with 1 or more and 3 or less carbons, X⁻is an anion, preferably an anion selected from a halogen ion and an alkyl sulfate ion with 1 or more and 3 or less carbons and more preferably an anion selected from a chloride ion, a bromide ion, an iodide ion, a methyl sulfate ion, an ethyl sulfate ion and a propyl sulfate ion.
<5> The viral or bacterial inactivation agent composition according to any of <1> to <4>, wherein component (B) is an aliphatic alcohol with a melting point of preferably 25°C or less, more preferably 10°C or less and further preferably 0°C or less.
<6> The viral or bacterial inactivation agent composition according to any of <1> to <5>, wherein component (B) is a compound represented by the following general formula (b1), 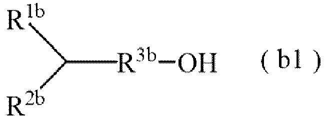 wherein R^{1b} is an alkyl group with 1 or more and 22 or less carbons, R^{2b} is an alkyl group with 1 or more and 22 or less carbons, R^{3b} is an alkylene group with 1 or more and 2 or less carbons, and a total carbon number of R^{1b}, R^{2b} and R^{3b} is 13 or more and 24 or less carbons.
<7> The viral or bacterial inactivation agent composition according to <6>, wherein in the general formula (b1), R^{1b} is an alkyl group with 1 or more, preferably 4 or more, preferably 8 or more, more preferably 9 or more and further preferably 10 or more, and 22 or less, preferably 18 or less, more preferably 14 or less and further preferably 12 or less carbons, R^{2b} is an alkyl group with 1 or more, preferably 4 or more and more preferably 8 or more, and 22 or less, preferably 18 or less, more preferably 14 or less and further preferably 10 or less carbons, a total carbon number of R^{1b} and R^{2b} is 11 or more, preferably 13 or more, more preferably 14 or more, further preferably 15 or more, furthermore preferably 16 or more and furthermore preferably 17 or more, and 22 or less, preferably 20 or less and more preferably 18 or less, R^{3b} is an alkylene group with 1 or more and 2 or less and preferably one carbon, and a total carbon number of R^{1b}, R^{2b} and R^{3b} is 13 or more, preferably 16 or more, more preferably 18 or more and further preferably 19 or more, and 24 or less, preferably 22 or less and more preferably 20 or less.
<8> The viral or bacterial inactivation agent composition according to any of <1> to <7>, wherein component (B) is one or more selected from branched C13-OH (an aliphatic alcohol with 13 carbons having a branched alkyl group), branched C16-OH (an aliphatic alcohol with 16 carbons having a branched alkyl group), branched C15-OH (an aliphatic alcohol with 15 carbons having a branched alkyl group), branched C17-OH (an aliphatic alcohol with 17 carbons having a branched alkyl group), branched C18-OH (an aliphatic alcohol with 18 carbons having a branched alkyl group), branched C19-OH (an aliphatic alcohol with 19 carbons having a branched alkyl group) and branched C20-OH (an aliphatic alcohol with 20 carbons having a branched alkyl group), preferably one or more selected from branched C16-OH, branched C18-OH and branched C20-OH, more preferably one or more selected from branched C18-OH and branched C20-OH and further preferably branched C20-OH.
<9> The viral or bacterial inactivation agent composition according to any of <1> to <8>, wherein a content of component (A) in the viral or bacterial inactivation agent composition is preferably 1 ppm or more, more preferably 5 ppm or more, further preferably 20 ppm or more and furthermore preferably 50 ppm or more, and preferably 5000 ppm or less, more preferably 3000 ppm or less, further preferably 1000 ppm or less, furthermore preferably 300 ppm or less and furthermore preferably 100 ppm or less.
<10> The viral or bacterial inactivation agent composition according to any of <2> to <9>, wherein the viral or bacterial inactivation composition contains components (a1) and (a2) as component (A), and a mass ratio of a content of component (a2) to a content of component (a1) in the composition, (a2)/(a1), is preferably 0/10 or more, more preferably 1.5/8.5 or more and further preferably 2/8 or more, and preferably 8/2 or less, more preferably 7/3 or less, further preferably 6/4 or less and furthermore preferably 4/6 or less.
<11> The viral or bacterial inactivation agent composition according to any of <1> to <10>, wherein a content of component (B) in the viral or bacterial inactivation agent composition is preferably 1 ppm or more, more preferably 2 ppm or more and further preferably 3 ppm or more, and preferably 1500 ppm or less, more preferably 1000 ppm or less, further preferably 500 ppm or less, furthermore preferably 250 ppm or less and furthermore preferably 100 ppm or less.
<12> The viral or bacterial inactivation agent composition according to any of <1> to <11>, wherein a mass ratio of a content of component (B) to a content of component (A) in the viral or bacterial inactivation agent composition, (B)/(A), is preferably 1/10 or more, more preferably 1/8 or more, further preferably 1/4 or more, furthermore preferably 1/2 or more and furthermore preferably 1/1 or more, and preferably 5/1 or less, more preferably 4/1 or less and further preferably 2/1 or less.
<13> The viral or bacterial inactivation agent composition according to any of <1> to <12>, wherein the composition further contains (C) a surfactant [excluding component (A)] [hereinafter referred to as component (C)].
<14> The viral or bacterial inactivation agent composition according to <13>, wherein component (C) is one or more selected from (C1) an anionic surfactant [hereinafter referred to as component (C1)], (C2) a nonionic surfactant [hereinafter referred to as component (C2)], (C3) a semipolar surfactant [hereinafter referred to as component (C3)] and (C4) an amphoteric surfactant [hereinafter referred to as component (C4)].
<15> The viral or bacterial inactivation agent composition according to any of <1> to <14>, wherein the composition further contains (D) a water-soluble organic solvent [excluding component (B)] [hereinafter referred to as component (D)].
<16> The viral or bacterial inactivation agent composition according to any of <1> to <15>, wherein the composition further contains (E) an organic acid or a salt thereof [hereinafter referred to as component (E)].
<17> The viral or bacterial inactivation agent composition according to any one of <1> to <16>, wherein the composition is used for the inactivation of an enveloped virus or a gram-negative bacterium.
<18> The viral or bacterial inactivation agent composition according to <17>, wherein the enveloped virus is one or more enveloped viruses selected from herpesvirus, influenza virus, paramyxovirus, rabies virus, respiratory syncytial virus, coronavirus, HIV, smallpox virus, hepatitis B virus, hepatitis C virus, hepatitis D virus, rubella virus, SARS coronavirus (SARS-CoV), SARS coronavirus 2 (SARS-CoV-2) and MERS coronavirus (MERS-CoV) and phage virus.
<19> The viral or bacterial inactivation agent composition according to <17> or <18>, wherein the gram-negative bacterium is one or more gram-negative bacteria selected from a bacterium of the genus *Moraxella,* a bacterium of the genus *Micrococcus,* a fungus of the genus *Saccharomyces,* the genus *Rhodotorula* or the genus *Pichia, Pseudomonas aeruginosa,* a bacterium of the genus *Pseudomonas, Escherichia coli, Alcaligenes faecalis, Klebsiella pneumoniae, Proteus vulgaris, Serratia marcescens* and *Methylobacterium.*
<20> A method for inactivating a virus or a bacterium present on a surface of interest including, bringing the viral or bacterial inactivation agent composition according to any of <1> to <19> or a treatment liquid obtained by diluting the same with water into contact with the surface of interest.
<21> The method for inactivating a virus or a bacterium according to <20>, wherein the virus or bacterium is an enveloped virus or a gram-negative bacterium.
<22> The method for inactivating a virus or a bacterium according to <21>, wherein the enveloped virus is one or more enveloped viruses selected from herpesvirus, influenza virus, paramyxovirus, rabies virus, respiratory syncytial virus, coronavirus, HIV, smallpox virus, hepatitis B virus, hepatitis C virus, hepatitis D virus, rubella virus, SARS coronavirus (SARS-CoV), SARS coronavirus 2 (SARS-CoV-2) and MERS coronavirus (MERS-CoV) and phage virus.
<23> The method for inactivating a bacterium according to <21> or <22>, wherein the gram-negative bacterium is one or more gram-negative bacteria selected from a bacterium of the genus *Moraxella,* a bacterium of the genus *Micrococcus,* a fungus of the genus *Saccharomyces,* the genus *Rhodotorula* or the genus *Pichia, Pseudomonas aeruginosa,* a bacterium of the genus *Pseudomonas, Escherichia coli, Alcaligenes faecalis, Klebsiella pneumoniae, Proteus vulgaris, Serratia marcescens* and *Methylobacterium.*
<24> The method for inactivating a virus or a bacterium according to any of <20> to <23>, wherein the surface of interest is a hard surface.
<25> The method for inactivating a virus or a bacterium according to any of <20> to <23>, wherein the surface of interest is of a livestock animal or livestock breeding equipment.
<26> The method for inactivating a virus or a bacterium according to any of <20> to <25>, wherein the viral or bacterial inactivation agent composition or the treatment liquid is sprayed or applied to the surface of interest on which a virus or a bacterium of interest is present or considered to be present.
<27> The method for inactivating a virus or a bacterium according to any of <20> to <25>, wherein the surface of interest on which a virus or a bacterium of interest is present or considered to be present is immersed in the viral or bacterial inactivation agent composition or the treatment liquid.
<28> The method for inactivating a virus or a bacterium according to any of <20> to <25>, wherein nonwoven fabric is impregnated with the viral or bacterial inactivation agent composition or the treatment liquid and brought into contact with the surface of interest on which a virus or a bacterium of interest is present or considered to be present.
<29> The method for inactivating a virus or a bacterium according to any of <20> to <28>, wherein a use amount of the viral or bacterial inactivation agent composition or the treatment liquid in terms of an amount of component (A) per cm² of a unit area of an object is preferably 0.05 µg/cm² or more and more preferably 1 µg/cm² or more, and preferably 15 mg/cm² or less and more preferably 1 mg/cm² or less.
<30> The method for inactivating a virus or a bacterium according to any of <20> to <29>, wherein a period of time during which the viral or bacterial inactivation agent composition or the treatment liquid is brought into contact with the surface of interest on which a virus or a bacterium of interest is present (a period of time during which they are left alone) is preferably 10 seconds or more, more preferably 1 minute or more and further preferably 5 minutes or more, and preferably 60 minutes or less, more preferably 30 minutes or less and further preferably 20 minutes or less.

### Examples

### <Formulation component>

In examples and comparative examples, the components below were used.
<Component (A)>
   · BAC: alkylbenzyldimethylammonium chloride, a compound of the general formula (a2) in which R^{5a} is an alkyl group with 8 to 18 carbons, R^{6a} and R^{7a} are methyl groups, and X⁻ is a chloride ion, component (a2), SANISOL C (effective amount 50%), manufactured by Kao Corporation
   · DDAC: didecyldimethylammonium chloride, a compound of the general formula (a1) in which R^{1a} and R^{2a} are alkyl groups with 10 carbons, R^{3a} and R^{4a} are methyl groups, and X⁻ is a chloride ion, component (a1), QUARTAMIN D-10P (effective amount 75%), manufactured by Kao Corporation
   · ADEBAC/DDAC: ethylbenzyl dimethyl ammonium chloride/dialkyl dimethyl ammonium chloride = mass ratio 4/6, Bardac 205M (effective amount 50%), manufactured by Lonza K.K.
<Component (B)>
   · Branched C16-OH: 2-hexyl decanol, a compound of the general formula (b1) in which R^{1b} is an alkyl group with 8 carbons, R^{2b} is an alkyl group with 6 carbons, and R^{3b} is an alkylene group with one carbon, ISOFOL 16, manufactured by Sasol Limited, melting point -15°C
   • Branched C18-OH: 2-octyl decanol, a compound in which R^{1b} is an alkyl group with 8 carbons, R^{2b} is an alkyl group with 8 carbons, and R^{3b} is an alkylene group with one carbon, ISOFOL 18E, manufactured by Sasol Limited
   · Branched C20-OH: 2-octyl dodecanol, a compound in which R^{1b} is an alkyl group with 10 carbons, R^{2b} is an alkyl group with 8 carbons, and R^{3b} is an alkylene group with one carbon, KALCOL 200GD, manufactured by Kao Corporation, melting point -7°C
   · Branched C13-OH: a branched alcohol with 13 carbons obtained in synthesis example (1) described later, a compound in which R^{1b} is an alkyl group, R^{2b} is an alkyl group, a total carbon number of R^{1b} and R^{2b} is 11, and R^{3b} is an alkylene group with one carbon
   · Branched C15-OH: a branched alcohol with 15 carbons obtained in synthesis example (2) described later, a compound in which R^{1b} is an alkyl group, R^{2b} is an alkyl group, a total carbon number of R^{1b} and R^{2b} is 13, and R^{3b} is an alkylene group with one carbon, melting point -26°C
   · Branched C17-OH: a branched alcohol with 17 carbons obtained in synthesis example (3) described later, a compound in which R^{1b} is an alkyl group, R^{2b} is an alkyl group, a total carbon number of R^{1b} and R^{2b} is 15, and R^{3b} is an alkylene group with one carbon, melting point -7°C
   · Branched C19-OH: a branched alcohol with 19 carbons obtained in synthesis example (4) described later, a compound in which R^{1b} is an alkyl group, R^{2b} is an alkyl group, a total carbon number of R^{1b} and R^{2b} is 17, and R^{3b} is an alkylene group with one carbon
<Component (C)>
   · Lauryldimethylamine oxide: a compound of the general formula (c3) in which R^{1c} is an alkyl group with 12 carbons, R^{2c} and R^{3c} are methyl groups, and p and q are 0, component (C3), AMPHITOL 20N (effective amount 35%), manufactured by Kao Corporation
   · Lauryl dimethyl aminoacetic acid betaine: a compound of the general formula (c4) in which R^{4c} is an alkyl group with 12 carbons, r is 0, R^{6c} and R^{7c} are methyl groups, R^{8c} is a methylene group, and B is -COO⁻, component (C4), AMPHITOL 24B (effective amount 27%), manufactured by Kao Corporation
   · Pentaerythritol propoxylate: AGRISOL F-100, manufactured by Kao Corporation
<Component (D)>
   · Ethanol: manufactured by FUJIFILM Wako Pure Chemical Corporation, component (D4)
   · Isopropanol: manufactured by FUJIFILM Wako Pure Chemical Corporation, component (D4)
<Component (E)>
   · Na gluconate: sodium gluconate, manufactured by FUJIFILM Wako Pure Chemical Corporation
   · Na citrate: sodium citrate, manufactured by FUJIFILM Wako Pure Chemical Corporation

### · Synthesis example (1)

### [Preparation of internal olefin]

3.0 kg of 1-dodecene (manufactured by Sigma-Aldrich Japan) as a raw material olefin and 30.0 g (1 mass% relative to the raw material olefin) of β-type zeolite (manufactured by Zeolyst International, product name: CP814E) as a catalyst were prepared in a four-neck flask, and reactions were carried out for 8 hours while nitrogen was allowed to flow at 165°C. The catalyst of the reaction liquid was filtered, and distillation purification was carried out to obtain an internal olefin with 12 carbons.

### [Preparation of branched alcohol]

2.0 kg of the obtained internal olefin with 12 carbons, 8.0 L of toluene (manufactured by FUJIFILM Wako Pure Chemical Corporation) as a solvent, 10.0 g of Rh(CO)₂(acac) (manufactured by Sigma-Aldrich Japan) as a catalyst and 120.0 mL of tricyclohexylphosphine (manufactured by Sigma-Aldrich Japan, 20% toluene solution) were prepared in an autoclave, and reactions were carried out for 5 hours at 110°C under CO/H₂ pressurization at 2.5 MPa. The solvent of the reaction liquid was evaporated to obtain an aldehyde with 13 carbons.

1.2 kg of the obtained aldehyde with 13 carbons and 6.5 L of ethanol (manufactured by FUJIFILM Wako Pure Chemical Corporation) were prepared, 327.0 g of sodium borohydride (manufactured by FUJIFILM Wako Pure Chemical Corporation) was added dividedly at 0°C, and reactions were carried out for 2 hours while nitrogen was allowed to flow at a room temperature. Next, the reaction liquid was added to 30 L of cold water, and reactions were carried out for 30 minutes. After 18 L of diisopropyl ether (manufactured by FUJIFILM Wako Pure Chemical Corporation) was added to the reaction liquid, and the solvent of an obtained organic layer was evaporated, distillation purification was carried out to obtain branched C13-OH.

### · Synthesis example (2)

Branched C15-OH was obtained in the same manner as in synthesis example (1) except that the raw material olefin was changed to 1-tetradecene.

### · Synthesis example (3)

Branched C17-OH was obtained in the same manner as in synthesis example (1) except that the raw material olefin was changed to 1-hexadecene.

### · Synthesis example (4)

Branched C19-OH was obtained in the same manner as in synthesis example (1) except that the raw material olefin was changed to 1-octadecene.

### [Examples 1 to 21 and comparative example 1]

### [Bacterial inactivation test method 1]

Bacterial inactivation test method 1 is described below.

### Preparation of test liquid and control liquid

BAC in an amount to attain an effective amount of 1 mass% after preparation was placed in a volumetric flask, and the flask was filled up to 10 mL with ethanol to prepare a 1 mass% solution of BAC.

DDAC in an amount to attain an effective amount of 1 mass% after preparation was placed in a 10 mL volumetric flask, and the flask was filled up to 10 mL with ethanol to prepare a 1 mass% solution of DDAC.

ADEBAC/DDAC in an amount to attain an effective amount of 1 mass% after preparation was placed in a 100 mL volumetric flask, and the flask was filled up to 100 mL with ethanol to prepare a 1000 ppm solution of ADEBAC/DDAC.

100 mg of each component (B) was placed in a 100 mL volumetric flask, and the flask was filled up to 100 mL with ethanol to prepare a 1000 ppm solution of component (B).

92.24 g of calcium chloride dihydrate (manufactured by FUJIFILM Wako Pure Chemical Corporation) and 54.58 g of magnesium chloride hexahydrate (manufactured by FUJIFILM Wako Pure Chemical Corporation) were dissolved in 1 L of sterile ion exchange water to prepare 5000°DH water.

Ion exchange water was sterilized by an autoclaving process (121°C × 20 min), and adjusted to have a pH of 7 with 1N hydrochloric acid (manufactured by KANTO CHEMICAL CO., INC.) to prepare sterile ion exchange water.

The 1000 ppm solution of BAC, the 1000 ppm solution of DDAC, the 1000 ppm solution of ADEBAC/DDAC, the 1000 ppm solution of each component (B) and the sterile ion exchange water were added to attain the formulation composition of each test liquid in Tables 1 and 2, and the 5000°DH water was also added to attain a hardness of 8°DH, thus preparing the test liquids in Tables 1 and 2. Note that all the formulation amounts of the components in each test liquid in Tables 1 and 2 are effective amounts.

The prepared test liquids were each dispensed to a sterile test tube in an amount of 5 mL, and the sterile ion exchange water was also dispensed to a sterile test tube in an amount of 5 mL as a control liquid. They were all left to stand at a room temperature until they were used for the test.

### (2) Bacterium used for test

### Escherichia coli NRBC3972 (National Institute of Technology and Evaluation)

### (3) Preparation of bacterial liquid used for test

One scrape was taken from a glycerol stock of the bacterium stored at -80°C in 10% glycerol (manufactured by FUJIFILM Wako Pure Chemical Corporation) to an SCD liquid culture medium (the SCD culture medium "Daigo" manufactured by NIHON PHARMACEUTICAL CO., LTD.), and cultured overnight at 37°C. The bacterial liquid after culture was subjected to centrifugation (5 minutes, 4500 g, 4°C) to remove a supernatant, and thereafter suspended by adding 10 ml of normal saline. After the centrifugation and supernatant removal operation was repeated in the same manner twice, an adjustment to OD600 = 0.8 was made in normal saline using a turbidity meter (Biowave Cell Density Meter CO8000, manufactured by BIOCHROM) such that the bacterium amount was 10⁹ cfu/mL.

### (4) Test operation

1. 50 µL of the bacterial liquid used for test was added to 5 mL of each of the test liquids or control liquid, immediately stirred with a vortex mixer for 15 seconds, and then, stirred within Bio Shaker (SIC-320HX, manufactured by AS ONE CORPORATION) (150 rpm/25°C).
2. After 5 minutes passed from the contact (addition of the bacterial liquid used for test), 500 µL was added to a sterile test tube to which 4.5 mL of a lecithin-polysorbate (LP) diluent (the LP diluent "Daigo" manufactured by NIHON PHARMACEUTICAL CO., LTD.) was dispensed in advance. The mixed liquid was stirred with a vortex mixer for 10 seconds. Further, 10-fold step-wise dilutions were made with the LP diluent.
3. 100 µL of a step-wise dilution at each step was smeared on an SCD agar culture medium (manufactured by NIHON PHARMACEUTICAL CO., LTD.), and cultured overnight at 37°C.
4. A number of colonies formed on the agar culture medium after culture was counted to calculate a number of viable bacteria.

### (5) Calculation of bacterium reduction amount

A bacterium reduction amount was calculated from ΔLOG (cfu/mL), a difference between logarithms of numbers of viable bacteria of the control liquid and each test liquid. The results are shown in Tables 1 and 2. It is considered that a test liquid with a larger ΔLOG (cfu/mL) has a higher bacterial inactivation effect.

### [Storage stability evaluation]

Each test liquid in Tables 1 and 2 was filled into a glass bottle and left to stand for 1 month at 4°C in a thermostatic chamber, and an appearance of the test liquid was visually observed and evaluated in accordance with the criteria below. The results are shown in Tables 1 and 2.
3: transparent liquid form
2: transparent liquid form, but precipitates can be slightly observed
1: turbid, crystalized or gelled

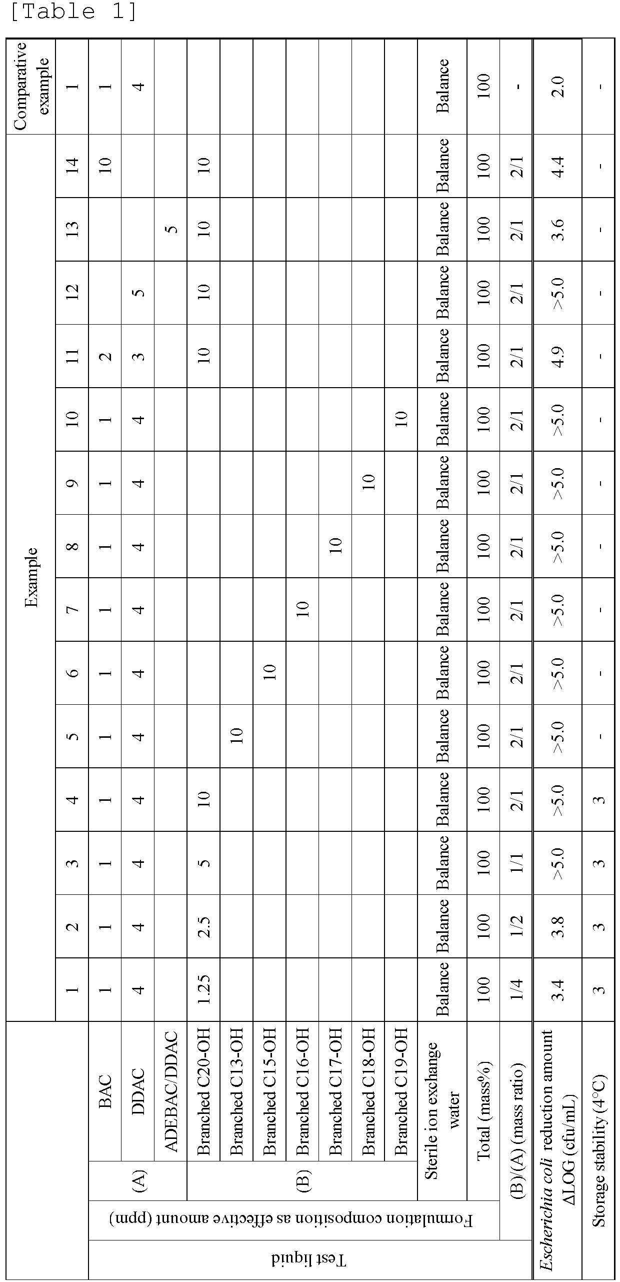

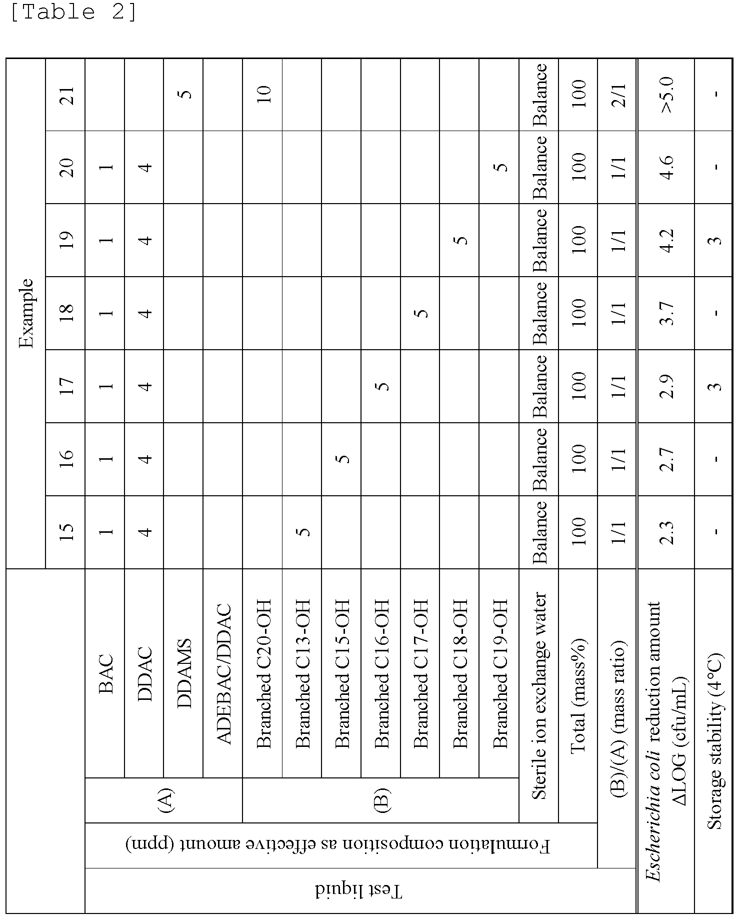

In Tables 1 and 2, when a bacterium reduction amount ΔLOG (cfu/mL) is expressed as ">5.0," it means that the bacterium was reduced to an amount equal to or less than the bacterium detection limit.

### [Examples 22 to 25 and comparative examples 2 to 3]

### [Viral inactivation test method]

A method for testing viral inactivation is described below.

### (1) Preparation of test liquid and control liquid

BAC in an amount to attain an effective amount of 1 mass% after preparation was placed in a volumetric flask, and the flask was filled up to 10 mL with ethanol to prepare a 1 mass% solution of BAC.

DDAC in an amount to attain an effective amount of 1 mass% after preparation was placed in a 10 mL volumetric flask, and the flask was filled up to 10 mL with ethanol to prepare a 1 mass% solution of DDAC.

0.5 g of branched C20-OH was placed in a 10 mL volumetric flask, and the flask was filled up to 10 mL with ethanol to prepare a 5 mass% solution of branched C20-OH.

92.24 g of calcium chloride dihydrate (manufactured by FUJIFILM Wako Pure Chemical Corporation) and 54.58 g of magnesium chloride hexahydrate (manufactured by FUJIFILM Wako Pure Chemical Corporation) were dissolved in 1 L of sterile ion exchange water to prepare 5000°DH water.

Ion exchange water was sterilized by an autoclaving process (121°C × 20 min), and adjusted to have a pH of 7 with 1N hydrochloric acid (manufactured by KANTO CHEMICAL CO., INC.) to prepare sterile ion exchange water.

In a vial, the 1 mass% solution of BAC, the 1 mass% solution of DDAC, the 5 mass% solution of branched C20-OH and the sterile ion exchange water were added to attain the formulation composition of each test liquid in Table 3, and the 5000°DH water was also added to attain a hardness of 8°DH, thus preparing the test liquids in Table 3. Note that all the formulation amounts of the components in each test liquid in Table 3 are effective amounts.

Further, 100 µL of 99.5% ethanol, 16 µL of the 5000°DH water and 9884 mL of the sterile ion exchange water were placed in a vial to prepare a control liquid.

The prepared test liquids and control liquid were each dispensed to a sterile tube in an amount of 45 µL, and all left to stand at a room temperature until they were used for the test.

### (2) Virus used for test

Influenza A virus (H1N1, A/PR/8/34) ATCC (American Type Culture Collection) VR-1469

### (3) Preparation of viral liquid used for test

After MDCK cells (cells derived from canine kidney, ATCC CCL-34, ATCC) were cultured in a T175 flask in a confluent state (2 × 10⁷ cells/flask), and washed with PBS (D-PBS(-)/pH 7.4, manufactured by Wako), the influenza A virus (H1N1, A/PR/8/34) in an amount to attain an MOI of 0.001 was added to 40 mL of a serum-free medium (Gibco, Hybridoma Serum Free Medium, PiN 123-00067, hereinafter referred to as SFM) in which acetyl trypsin (manufactured by Sigma) to attain a final concentration of 2 µg/mL and 50 mg/L gentamicin (gentamicin sulfate solution (50 mg/ml), manufactured by FUJIFILM Wako Pure Chemical Corporation) were added, and the cells were infected therewith. After about 48 hours of culture under 5% CO₂ at 37°C, a cell culture supernatant was collected, and the supernatant subjected to centrifugation (800 g/10 min at 4°C) was again subjected to centrifugation (13,000 g/10 min at 4°C) to remove a supernatant, thereby obtaining a virus concentrate (precipitation). The virus concentrate was resuspended in 150 to 200 µL of SFM, and thereafter further subjected to centrifugation (800 g/10 min at 4°C) to collect a supernatant, thereby obtaining a concentrated influenza A virus liquid. A viral titer of the concentrated influenza A virus liquid was measured by focus assays, and the liquid with the viral titer adjusted to 2 × 10⁸ FFU/mL on the basis of measurement results was used as a test viral liquid.

The viral titer by focus assays was measured by the method below.

After MDCK cells were cultured in a 12 well plate for focus assays to be confluent using an Eagle's minimal essential medium (MEM culture medium) in which 5% FBS was added, and washed with PBS, they were habituated to SFM for 30 minutes. The sample after reactions was added to the above MDCK cells cultured in the 12 well plate in an amount of 500 pL/well, and the cells were incubated for 30 minutes to be infected with the influenza virus. The present test was conducted by three-time measurements. After infection, a washing operation with SFM was performed, 1.2 w/v%-CEOLUS (Asahi Kasei Chemicals Corporation, RC591) and 2.0 pg/mL-acetylated trypsin (manufactured by Sigma)-containing SFM were added to be 2.0 mL/well, and culture was performed for 18 to 22 hours. After culture, the wells were washed with PBS three times, and then, 100% methanol (manufactured by Wako Corporation) was added to immobilize the cells. The immobilized cells were reacted with a primary antibody: Anti-NPantibody (mouse hybridoma (4E6) cell culture supernatant (which was actually used in J Virol. 2008; 82: 5940-50)) and a secondary antibody: HRP linked Goat Anti-Mouse IgG + IgM Antibody (manufactured by Jackson ImmunoResearch Laboratories Inc.), and reacted with HRP using a DEPDA reaction liquid (100 mM citrate buffer (pH 6.0) containing 1.2 mM N,N-diethyl-p-phenylenediamine dihydrochloride (manufactured by FUJIFILM Wako Pure Chemical Corporation), 0.003% hydrogen peroxide and 2 mM 4-chloro-1-naphthol (manufactured by FUJIFILM Wako Pure Chemical Corporation)), and a number of dyed focuses was counted. The focus assays were performed by independent three-time measurements. The value was expressed as an average value of results of the three-time measurements.

### (4) Preparation of diluent for measurements (SCDLP solution)

38 g of SCDLP powder (manufactured by NIHON PHARMACEUTICAL CO., LTD., PiN 395-00265) was weighed and dissolved in 1 L of ion exchange water, and thereafter subjected to an autoclaving process (121°C × 20 min).

### (5) Test operation

1. 5 µL of the viral liquid used for test was added to 45 µL of each of the test liquids in Table 3 or control liquid, immediately stirred with a vortex mixer for 15 seconds, and then left to stand.
2. After 5 minutes passed from the contact (addition of the viral liquid used for test), 950 µL of SCDLP was added and stirred with a vortex mixer for 10 seconds. Further, 10-fold step-wise dilutions were made with SFM.
3. 500 µL of each liquid diluted with SFM was inoculated into MDCK cells in a confluent state in a 12 well plate, 3 wells per liquid.
4. 18 to 22 hours later, an infectious titer (FFU/mL) of the inoculated solution was measured by the above focus assay method.

### (5) Calculation of virus reduction amount

A virus reduction amount was calculated from ΔLOG (FFU/mL), a difference between logarithms of infectious titers of the control liquid and each test liquid. The results are shown in Table 3. It is considered that a test liquid with a larger ΔLOG (FFU/mL) has a higher viral or bacterial inactivation effect.

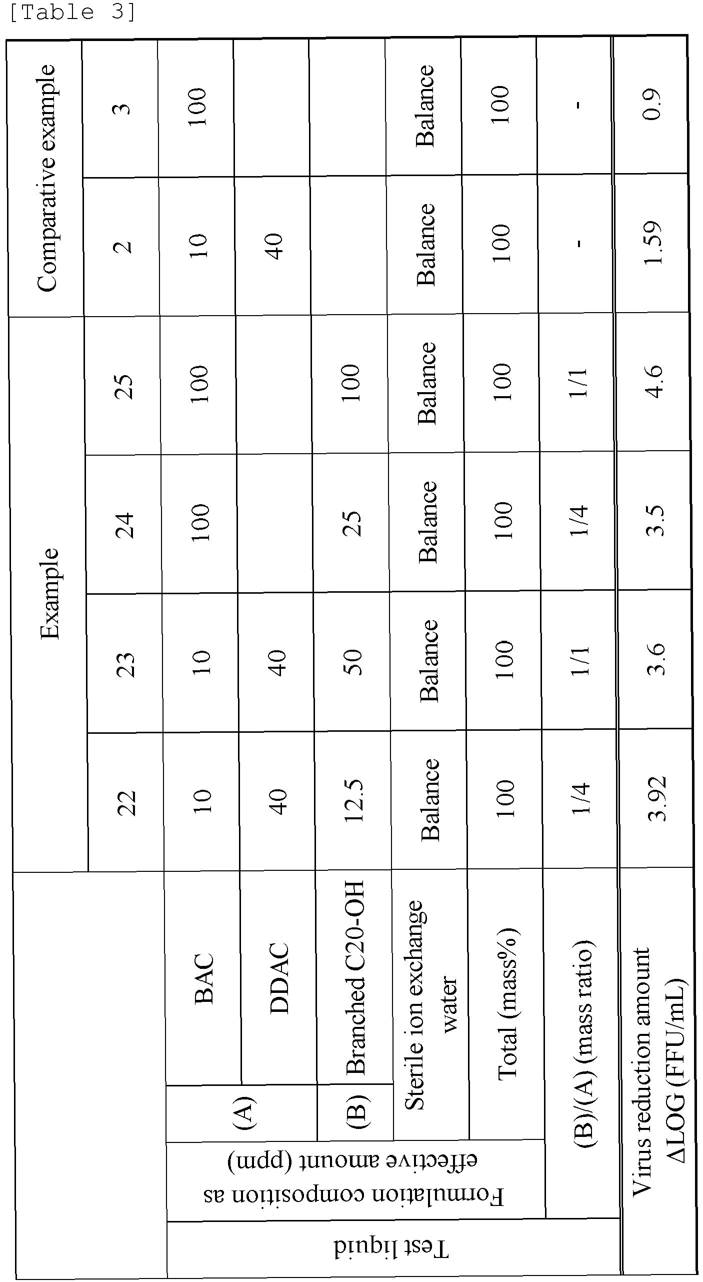

### [Examples 26 to 33]

### [Bacterial inactivation test method 2]

Bacterial inactivation test method 2 is described below.

### (1) Preparation of test liquid and control liquid

### · Examples 26 to 28

DDAMS in an effective amount of 2.5 g, 0.31 g of branched C20-OH, lauryldimethylamine oxide in an effective amount of 1.25 g, lauryl dimethyl aminoacetic acid betaine in an effective amount of 3.75 g, 0.38 g of Na gluconate, 0.05 g of Na citrate and 3.75 g of ethanol were mixed to prepare a formulation liquid. 0.026 g of the formulation liquid was diluted with 4.977 g of sterile ion exchange water to obtain the test liquid of example 26.

0.052 g of the formulation liquid prepared in example 26 was diluted with 4.948 g of sterile ion exchange water to obtain the test liquid of example 27.

0.104 g of the formulation liquid prepared in example 26 was diluted with 4.896 g of sterile ion exchange water to obtain the test liquid of example 28.

### · Example 29

DDAMS in an effective amount of 2.52 g, 0.31 g of branched C20-OH, lauryldimethylamine oxide in an effective amount of 2.5 g, lauryl dimethyl aminoacetic acid betaine in an effective amount of 7.5 g, 0.38 g of Na gluconate, 0.05 g of Na citrate and 3.75 g of ethanol were mixed to prepare a formulation liquid. 0.026 g of the formulation liquid was diluted with 4.977 g of sterile ion exchange water to obtain the test liquid of example 29.

### · Examples 30 to 32

DDAMS in an effective amount of 10 g, 1.25 g of branched C20-OH, 10 g of pentaerythritol propoxylate, 0.75 g of Na gluconate, 0.10 g of Na citrate, 3.5 g of isopropanol and 20.26 g of sterile ion exchange water were mixed to prepare a formulation liquid. 0.025 g of the formulation liquid was diluted with 4.975 g of sterile ion exchange water to obtain the test liquid of example 30.

0.208 g of the formulation liquid prepared in example 30 was diluted with 4.792 g of sterile ion exchange water to obtain the test liquid of example 31.

0.416 g of the formulation liquid prepared in example 30 was diluted with 4.584 g of sterile ion exchange water to obtain the test liquid of example 32.

### · Example 33

DDAMS in an effective amount of 5 g, 0.63 g of branched C20-OH, 20 g of pentaerythritol propoxylate, 0.37 g of Na gluconate, 0.05 g of Na citrate, 1.75 g of isopropanol and 20.13 g of sterile ion exchange water were mixed to prepare a formulation liquid. 0.05 g of the formulation liquid was diluted with 4.95 g of sterile ion exchange water to obtain the test liquid of example 33.

Note that all the formulation amounts of the components in each test liquid in Table 4 are effective amounts.

### (2) Bacterium used for test Moraxella osloensis (clothing isolate)

### (3) Preparation of bacterial liquid used for test

One scrape was taken from a glycerol stock of the bacterium stored at -80°C in 10% glycerol (manufactured by FUJIFILM Wako Pure Chemical Corporation) to an SCD liquid culture medium (the SCD culture medium "Daigo" manufactured by NIHON PHARMACEUTICAL CO., LTD.), and cultured overnight at 37°C. The bacterial liquid after culture was subjected to centrifugation (5 minutes, 4500 g, 4°C) to remove a supernatant, and thereafter suspended by adding 10 ml of normal saline. After the centrifugation and supernatant removal operation was repeated in the same manner twice, an adjustment to OD600 = 1 was made in normal saline using a turbidity meter (Biowave Cell Density Meter CO8000, manufactured by BIOCHROM) such that the bacterium amount was 10⁹ cfu/mL.

### (4) Test operation

1. 50 µL of the bacterial liquid used for test was added to 5 mL of each of the test liquids or control liquid, immediately stirred with a vortex mixer for 15 seconds, and then stirred within Bio Shaker (SIC-320HX, manufactured by AS ONE CORPORATION) (150 rpm/25°C).
2. After 5 minutes passed from the contact (addition of the bacterial liquid used for test), 500 µL was added to a sterile test tube to which 4.5 mL of a lecithin-polysorbate (LP) diluent (the LP diluent "Daigo" manufactured by NIHON PHARMACEUTICAL CO., LTD.) was dispensed in advance. The mixed liquid was stirred with a vortex mixer for 10 seconds. Further, 10-fold step-wise dilutions were made with the LP diluent.
3. 100 µL of a step-wise dilution at each step was smeared on an SCD agar culture medium (manufactured by NIHON PHARMACEUTICAL CO., LTD.), and cultured overnight at 37°C.
4. A number of colonies formed on the agar culture medium after culture was counted to calculate a number of viable bacteria.

### (5) Calculation of bacterium reduction amount

A bacterium reduction amount was calculated from ΔLOG (cfu/mL), a difference between logarithms of numbers of viable bacteria of the control liquid and each test liquid. The results are shown in Table 4. It is considered that a test liquid with a larger ΔLOG (cfu/mL) has a higher bacterial inactivation effect.

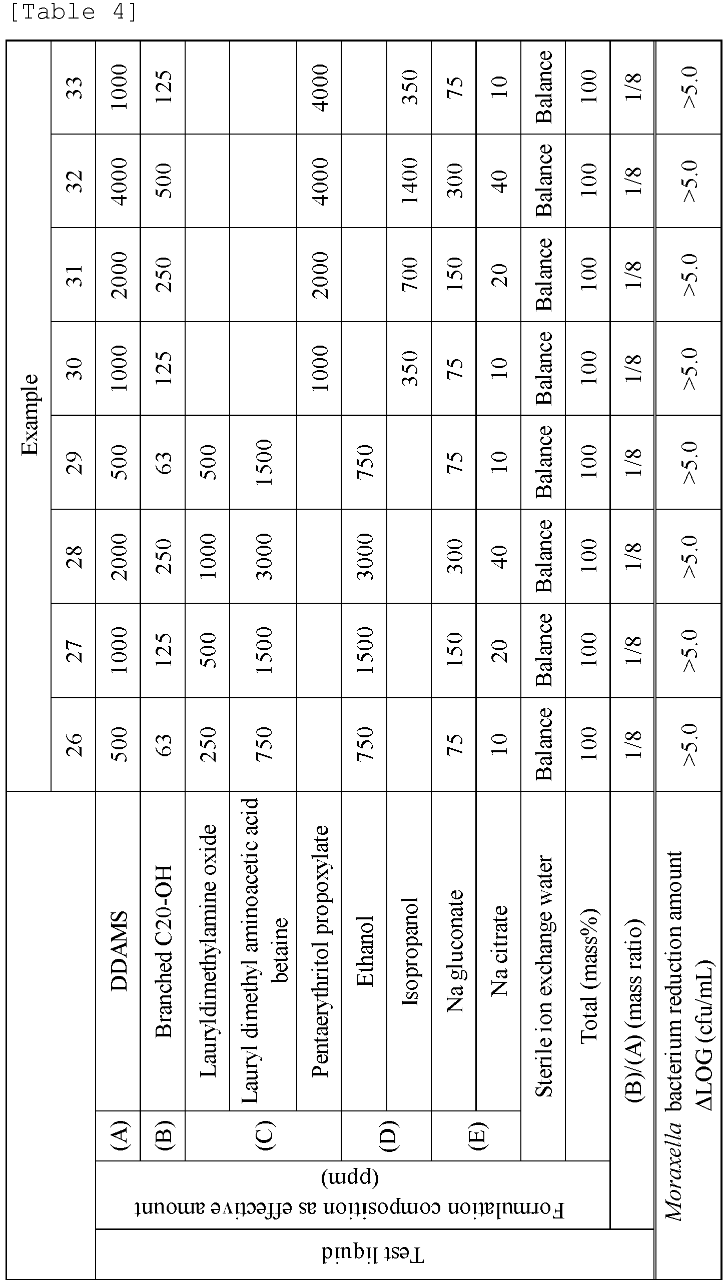

In Table 4, when a bacterium reduction amount ΔLOG (cfu/mL) is expressed as ">5.0," it means that the bacterium was reduced to an amount equal to or less than the bacterium detection limit.

### [Examples 34 to 36 and comparative examples 4 to 9]

### [Bacterial inactivation test method 3]

Bacterial inactivation test method 3 is described below.

### Preparation of test liquid and control liquid

BAC in an amount to attain an effective amount of 1000 ppm after preparation was placed in a 100 mL volumetric flask, and the flask was filled up to 100 mL with ethanol to prepare a 1000 ppm solution of BAC.

DDAC in an amount to attain an effective amount of 1000 ppm after preparation was placed in a 100 mL volumetric flask, and the flask was filled up to 100 mL with ethanol to prepare a 1000 ppm solution of DDAC.

100 mg of branched C20-OH was placed in a 100 mL volumetric flask, and the flask was filled up to 100 mL with ethanol to prepare a 1000 ppm solution of branched C20-OH.

92.24 g of calcium chloride dihydrate (manufactured by FUJIFILM Wako Pure Chemical Corporation) and 54.58 g of magnesium chloride hexahydrate (manufactured by FUJIFILM Wako Pure Chemical Corporation) were dissolved in 1 L of sterile ion exchange water to prepare 5000°DH water.

Ion exchange water was sterilized by an autoclaving process (121°C × 20 min), and adjusted to have a pH of 7 with 1N hydrochloric acid (manufactured by KANTO CHEMICAL CO., INC.) to prepare sterile ion exchange water.

The 1000 ppm solution of BAC, the 1000 ppm solution of DDAC, the 1000 ppm solution of branched C20-OH and the sterile ion exchange water were added to attain the formulation composition of each test liquid in Table 5, and the 5000°DH water was also added to attain a hardness of 8°DH, thus preparing the test liquids in Table 5. Note that all the formulation amounts of the components in each test liquid in Table 5 are effective amounts.

The prepared test liquids were each dispensed to a sterile test tube in an amount of 5 mL, and the sterile ion exchange water was dispensed to a sterile test tube in an amount of 5 mL as a control liquid. They were all left to stand at a room temperature until they were used for the test.

### (2) Bacteria used for test

· *Moraxella osloensis* (clothing isolate)
· *Micrococcus luteus* (clothing isolate)
· *Pseudomonas aeruginosa* NBRC 13736 (National Institute of Technology and Evaluation)

### (3) Preparation of bacterial liquids used for test

In example 34 and comparative examples 4 and 5, a bacterial liquid used for test was prepared in the same manner as in the method described in (3) of bacterial inactivation test method 1 except that the bacterium used for test was changed to *Moraxella osloensis.*

In example 35 and comparative examples 6 and 7, a bacterial liquid used for test was prepared in the same manner as in the method described in (3) of bacterial inactivation test method 1 except that the bacterium used for test was changed to *Micrococcus luteus.*

In example 36 and comparative examples 8 and 9, a bacterial liquid used for test was prepared in the same manner as in the method described in (3) of bacterial inactivation test method 1 except that the bacterium used for test was changed to *Pseudomonas aeruginosa.*

### (4) Test operation

In example 34 and comparative examples 4 and 5, a test operation was performed in the same manner as in the method described in (4) of bacterial inactivation test method 1 except that the bacterium used for test was changed to *Moraxella osloensis.*

In example 35 and comparative examples 6 and 7, a test operation was performed in the same manner as in the method described in (4) of bacterial inactivation test method 1 except that the bacterium used for test was changed to *Micrococcus luteus.*

In example 36 and comparative examples 8 and 9, a test operation was performed in the same manner as in the method described in (4) of bacterial inactivation test method 1 except that the bacterium used for test was changed to *Pseudomonas aeruginosa.*

### (5) Calculation of bacterium reduction amount

A bacterium reduction amount was calculated from ΔLOG (cfu/mL), a difference between logarithms of numbers of viable bacteria of the control liquid and each test liquid. The results are shown in Table 5. It is considered that a test liquid with a larger ΔLOG (cfu/mL) has a higher bacterial inactivation effect.

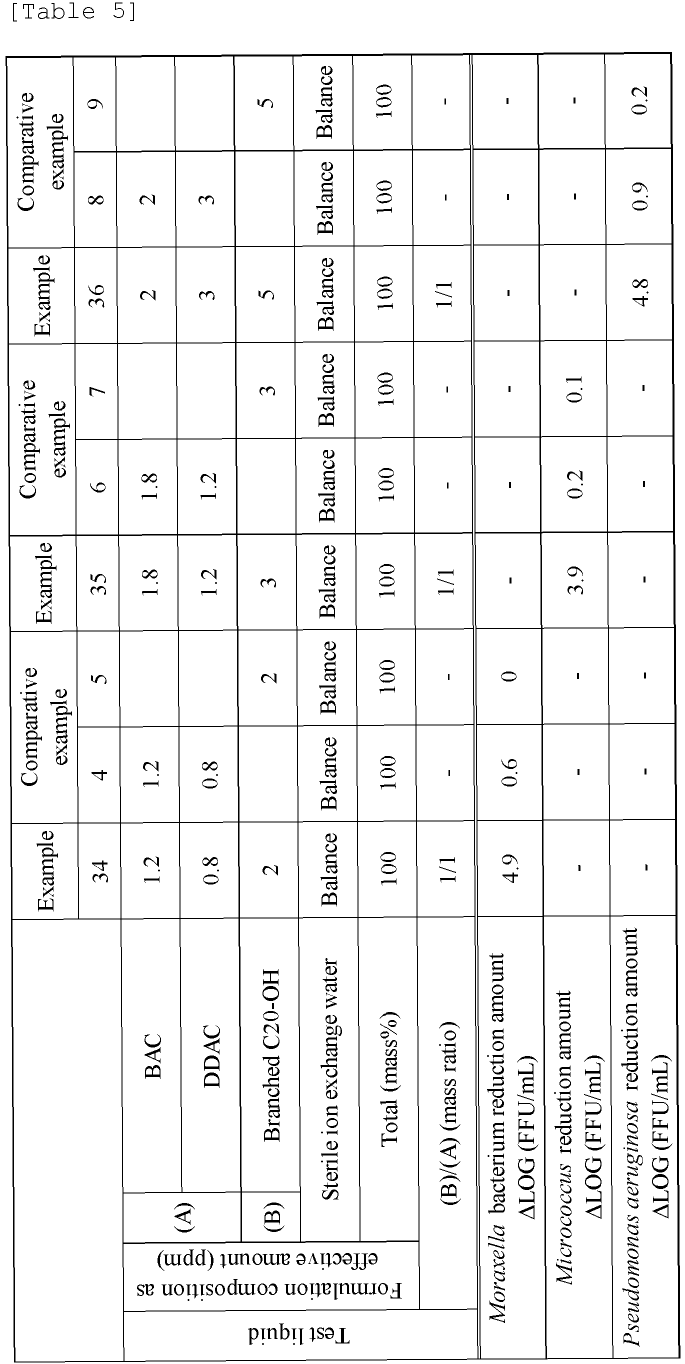

## Claims

1. A viral or bacterial inactivation agent composition comprising, (A) a quaternary ammonium salt-type surfactant (hereinafter referred to as component (A)), (B) an aliphatic alcohol with 8 or more and 24 or less carbons having a branched alkyl group (hereinafter referred to as component (B)) and water.

2. The viral or bacterial inactivation agent composition according to claim 1, wherein the component (A) is one or more selected from compounds represented by the following general formula (a1) and compounds represented by the following general formula (a2), 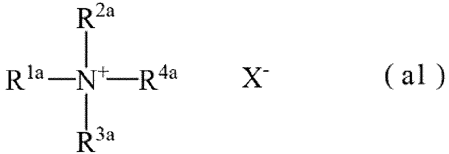
wherein R^{1a} is an aliphatic hydrocarbon group with 8 or more and 18 or less carbons, R^{2a} is a group selected from an aliphatic hydrocarbon group with 8 or more and 18 or less carbons, an alkyl group with 1 or more and 3 or less carbons and a hydroxyalkyl group with 1 or more and 3 or less carbons, R^{3a} and R^{4a} each independently represent a group selected from an alkyl group with 1 or more and 3 or less carbons and a hydroxyalkyl group with 1 or more and 3 or less carbons, and X- is an anion, and 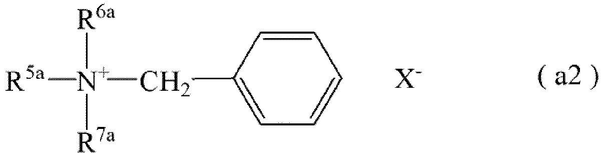
wherein R^{5a} is an aliphatic hydrocarbon group with 8 or more and 18 or less carbons, R^{6a} and R^{7a} each independently represent a group selected from an alkyl group with 1 or more and 3 or less carbons and a hydroxyalkyl group with 1 or more and 3 or less carbons, and X⁻ is an anion.

3. The viral or bacterial inactivation agent composition according to claim 1 or 2, wherein the component (B) is an aliphatic alcohol with a melting point of 25°C or less.

4. The viral or bacterial inactivation agent composition according to any one of claims 1 to 3, wherein the component (B) is a compound represented by the following general formula (b1), 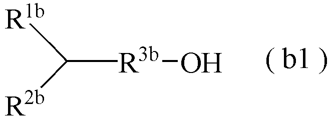 wherein R^{1b} is an alkyl group with 1 or more and 22 or less carbons, R^{2b} is an alkyl group with 1 or more and 22 or less carbons, R^{3b} is an alkylene group with 1 or more and 2 or less carbons, and a total carbon number of R^{1b}, R^{2b} and R^{3b} is 13 or more and 24 or less carbons.

5. The viral or bacterial inactivation agent composition according to any one of claims 1 to 4, wherein a mass ratio of a content of the component (B) to a content of the component (A), (B)/(A), is 1/8 or more and 5/1 or less.

6. The viral or bacterial inactivation agent composition according to any one of claims 1 to 5, wherein a content of the component (A) is 5 ppm or more and 1000 ppm or less.

7. The viral or bacterial inactivation agent composition according to any one of claims 1 to 6, wherein the composition is used for the inactivation of an enveloped virus or a gram-negative bacterium.

8. A method for inactivating a virus or a bacterium present on a surface of interest comprising, bringing the viral or bacterial inactivation agent composition according to any one of claims 1 to 6 or a treatment liquid obtained by diluting the same with water into contact with the surface of interest.

9. The method for inactivating a virus or a bacterium according to claim 8, wherein the virus or bacterium is an enveloped virus or a gram-negative bacterium.

10. The method for inactivating a virus or a bacterium according to claim 8 or 9, wherein the surface of interest is a hard surface.

11. The method for inactivating a virus or a bacterium according to claim 8 or 9, wherein the surface of interest is of a livestock animal or livestock breeding equipment.
